# EUROPEAN PATENT SPECIFICATION

(11) **EP 1 583 729 B1**
(45) Date of publication and mention of the grant of the patent: **06.06.2018**
(21) Application number: 04701106.9
(22) Date of filing: 09.01.2004
(51) Int. Cl.: C07C 41/36, C07C 41/40, C07C 41/26, C07C 43/23

(54) **METHOD OF PURIFYING REDUCED COENZYME Q**
VERFAHREN ZUR AUFREINIGUNG VON REDUZIERTEM COENZYM Q
METHODE DE PURIFICATION DE LA COENZYME Q REDUITE

(30) Priority: 10.01.2003 JP 2003005151
(43) Date of publication of application: 12.10.2005
(73) Proprietor: KANEKA CORPORATION, Osaka (JP)
(72) Inventor: UEDA, Takahiro, Kobe-shi, Hyogo 6550872 (JP); OHNO, Tadao, Kobe-shi, Hyogo 6550872 (JP); KITAMURA, Shiro, 6730882 (JP); UEDA, Yasuyoshi, 6711227 (JP)
(74) Representative: Hoffmann Eitle
(86) International application number: PCT/JP2004/000117
(87) International publication number: WO 2004/063131

(56) References cited:
- EP-A1- 1 408 024
- EP-A1- 1 415 970
- WO-A1-03/006409
- WO-A1-03/006411
- GB-A- 947 643
- JP-A- 53 133 687

## Description

### TECHNICAL FIELD

The present invention relates to a method of purifying reduced coenzyme Q₁₀. Reduced coenzyme Q₁₀ shows higher level of oral absorbability as compared with oxidized coenzyme Q₁₀ and is a compound useful as an ingredient in good foods, functional nutritive foods, specific health foods, nutritional supplements, nutrients, animal drugs, drinks, feeds, cosmetics, medicines, remedies, preventive drugs, etc.

### BACKGROUND ART

It is known that reduced coenzyme Q₁₀ can be prepared by producing coenzyme Q₁₀ in the conventional manner, for example by synthesis, fermentation, or extraction from natural products, and concentrating a reduced coenzyme Q₁₀-containing eluate fraction resulting from chromatography (c.f. JP-A-10-109933) . It is also described in the above-cited publication that, in this case, the chromatographic concentration may be carried out after reduction of oxidized coenzyme Q₁₀, occurred as an impurity in the reduced coenzyme Q₁₀, with a reducing agent such as sodium borohydride or sodium dithionite (sodium hyposulfite), or reduced coenzyme Q₁₀ may be prepared by reacting the reducing agent mentioned above with an existing highly pure grade of coenzyme Q₁₀. Also known are the method which comprises using zinc as a reducing agent (Journal of Labelled Compounds, vol. 6, 1970, 66-75), the method using sodium borohydride (GB947643)
and the method which comprises using vitamin C species (i.e. ascorbic acid or related compounds such as ascorbic acid, ascorbic acid palmitate, and ascorbic acid stearate) as reducing agents (WO 01/52822 A1).

However, the reduced coenzyme Q₁₀ produced in such a manner cannot always be recovered in a highly pure state. For example, it is often obtained in the form of low-purity crystals, semisolids or oil containing oxidized coenzyme Q₁₀ and other impurities. When crystals of reduced coenzyme Q₁₀ is recovered from an organic solvent solution containing reduced coenzyme Q₁₀, in particular, it is difficult to remove water-soluble impurities, particularly the reducing agent used for converting oxidized coenzyme Q₁₀ into reduced coenzyme Q₁₀ and/or impurities derived from such reducing agent and, therefore, the crystals obtained very often contain such water-soluble impurities and are of low purity.

Methods for producing a reduced coenzyme Q10 crystal by crystallizing reduced coenzyme Q10 in an aqueous solution (WO 03/6411 A1) or a solution of an alcohol and/or a ketone (WO 03/6409 A1) are state of the art in accordance with Art. 54(3) EPC. In these methods the crystals are isolated by centrifugation, pressure filtration, or vacuum filtration, if necessary followed by cake washing.

### DISCLOSURE OF THE INVENTION

In view of the foregoing, the present invention has an object to provide a purification method for removing impurities, in particular water-soluble impurities, contained in reduced coenzyme Q₁₀, and thereby producing high-quality reduced coenzyme Q₁₀ in a convenient and efficient manner on an industrial scale production.

As a result of intensive investigations, the present inventors found that when an attempt was made to remove the water-soluble impurities remaining in reduced coenzyme Q₁₀ by using water alone, particularly to remove the reducing agent and/or impurities derived from reducing agent, it was not always easy to decrease content of said impurities to at least to trace levels. It was also found that reduced coenzyme Q₁₀ showed very poor wettability characteristics against water and thus it was very difficult to obtain slurry thereof having good properties. It was found, however, that the water-soluble impurities, in particular the reducing agent and/or impurities derived therefrom, remaining in reduced coenzyme Q₁₀ could be removed efficiently with good operationality by washing reduced coenzyme Q₁₀ (crystals or oil) with a water-soluble organic solvent or a mixed solvent composed of a water-soluble organic solvent and water. Based on these findings, the present invention has now completed.

Thus, the present invention provides a method of purifying reduce coenzyme Q₁₀ wherein water-soluble impurities are removed from the reduced coenzyme Q10,
wherein washing of the crystals and/or oil of the reduced coenzyme Q₁₀ comprises bringing crystals and/or oil of the reduced coenzyme Q₁₀ into contact with a water-soluble organic solvent or a mixed solvent composed of a water-soluble organic solvent and water in a vessel and wherein the washing of the crystals and/or oil of reduced coenzyme Q₁₀ is carried out in a state of dispersion of the crystals and/or oil of reduced coenzyme Q₁₀ in the water-soluble organic solvent or the mixed solvent composed of the water-soluble organic solvent and water and
wherein the crystals and/or oil of reduced coenzyme Q₁₀ are/is selected from the group consisting of: crystals obtainable by crystallization from or concentration to dryness of a solution containing reduced coenzyme Q₁₀; a solid resulting from solidification of oil of reduced coenzyme Q₁₀; an oil resulting from melting of crystals of reduced coenzyme Q₁₀; and an oil obtainable by concentrating a reduced coenzyme Q₁₀-containing solution at a temperature not lower than the melting temperature.

In accordance with the method of the present invention, the water-soluble impurities contained in reduced coenzyme Q₁₀ can be conveniently and efficiently removed at least to a trace level and reduced coenzyme Q₁₀ of very high quality can be obtained as a crystalline or an oily form.

In purifying oil of reduced coenzyme Q₁₀, it is also possible to crystallize it by cooling together with the solvent used for washing to recover the crystals formed, or to solidify of reduced coenzyme Q₁₀ by contacting seed crystals to an oily form of reduced coenzyme Q₁₀ at a temperature lower than the melting point thereof, to recover the crystals formed.

### DETAILED DISCLOSURE OF THE INVENTION

In the following, the present invention is described in more detail.

The method of purifying reduced coenzyme Q₁₀ according to the present invention is a method comprises washing crystalline and/or an oily form of reduced coenzyme Q₁₀ with a water-soluble organic solvent or a mixed solvent composed of a water-soluble organic solvent and water to thereby remove a water-soluble impurity from the crystals and/or oil of reduced coenzyme Q₁₀.

Namely, in accordance with the invention, washing is carried out with a water-soluble organic solvent or a mixed solvent composed of a water-soluble organic solvent and water to conveniently and efficiently remove water-soluble impurities remaining in crystals and/or oil of reduced coenzyme Q₁₀, in particular the reducing agent and/or impurities derived from a reducing agent, which are to be mentioned later herein.

The water-soluble organic solvent used in the practice of the present invention is not particularly restricted provided that it is highly miscible with water, but includes alcohols, ethers, ketones, nitriles, amides, sulfur-containing compounds, fatty acids, and the like.

The alcohols may be used for the present invention are not particularly restricted but may be cyclic or acyclic, or saturated or unsaturated. Saturated ones are preferred, however. Generally, one containing 1 to 20 carbon atoms, preferably 1 to 12 carbon atoms, more preferably 1 to 6 carbon atoms, still more preferably 1 to 3 carbon atoms, and particularly preferably 2 or 3 carbon atoms, may favorably be used.

As specific examples of the alcohols, there may be mentioned methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol, 3,5,5-trimethyl-1-hexanol, 1-decanol, 1-undecanol, 1-dodecanol, allyl alcohol, propargyl alcohol, benzyl alcohol, cyclohexanol, 1-methylcyclohexanol, 2-methylcyclohexanol, 3-methylcyclohexanol, 4-methylcyclohexanol, 2-methoxyethanol, 2-ethoxyethanol, 2-(methoxymethoxy)ethanol, 2-isopropoxyethanol, 2-buthoxyethanol, 2-(isopentyloxy)ethanol, 2-(hexyloxy)ethanol, furfuryl alcohol, 1-methoxy-2-propanol, 1-ethoxy-2-propanol, 1,2-ethanediol, 1,2-propanediol, 1,3-propanediol, 1,2-butanediol, 1,3-butanediol, 1,4-butanediol, 2,3-butanediol, 1,5-pentanediol, 2-butane-1,4-diol, 2-methyl-2,4-pentanediol, 2-ethyl-1,3-hexanediol, diethylene glycol, triethylene glycol, tetraethylene glycol, polyethylene glycol, dipropylene glycol, polypropylene glycol, glycerol, etc.

As monohydric alcohols, preferred ones which may be mentioned are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, 1-heptanol, 2-heptanol, 3-heptanol, 1-octanol, 2-octanol, 2-ethyl-1-hexanol, 1-nonanol, 1-decanol, 1-undecanol, 1-dodecanol, benzyl alcohol, cyclohexanol, 1-methylcyclohexanol, 2-methylcyclohexanol, 3-methylcyclohexanol, 4-methylcyclohexanol, 2-methoxyethanol, 2-ethoxyetanol, 2-(methoxymethoxy)ethanol, etc. More preferred are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, 1-hexanol, 2-methyl-1-pentanol, 4-methyl-2-pentanol, 2-ethyl-1-butanol, cyclohexanol, etc. Still more preferred are methanol, ethanol, 1-propanol, 2-propanol, 1-butanol, 2-butanol, isobutyl alcohol, tert-butyl alcohol, 1-pentanol, 2-pentanol, 3-pentanol, 2-methyl-1-butanol, isopentyl alcohol, tert-pentyl alcohol, 3-methyl-2-butanol, neopentyl alcohol, etc. Particularly preferred are methanol, ethanol, 1-propanol, 2-propanol, 1-butanol, 2-butanol, isobutyl alcohol, 2-methyl-1-butanol, isopentyl alcohol, etc. Further preferred are methanol, ethanol, 1-propanol, 2-propanol, etc., and most preferred is ethanol.

As the dihydric alcohol, preferred ones which may be mentioned are 1,2-ethanediol, 1,2-propandiol, 1,3-propandiol, 2-butane-1,4-diol, 2-methyl-2,4-pentanediol, 2-ethyl-1,3-hexanediol, diethylene glycol, triethylene, glycol, tetraethylene glycol, polyethylene glycol, dipropylene glycol, polypropylene glycol, etc., and most preferred are 1,2-propanediol and polyethylene glycol.

As the trihydric alcohol, glycerol may be preferably used.

The ethers are not particularly restricted, and may be cyclic or acyclic, or saturated or unsaturated. But in general, saturated ones are preferably used. Generally, ones containing 3 to 20 carbon atoms, and preferably 4 to 12 carbon atoms and more preferably 4 to 8 carbon atoms are used.

As specific examples, there may be mentioned, for example, diethyl ether, methyl tert-butyl ether, dipropyl ether, diisopropyl ether, dibutyl ether, dihexyl ether, ethyl vinyl ether, butyl vinyl ether, anisol, phenetole, butyl phenyl ether, methoxytoluene, dioxane, furan, 2-methylfuran, tetrahydrofuran, tetrahydropyran, ethylene glycol dimethyl ether, ethylene glycol diethyl ether, ethylene glycol dibutyl ether, ethylene glycol monomethyl ether, ethylene glycol monoethyl ether, ethylene glycol monobutyl ether, diethylene glycol monomethyl ether, diethylene glycol monoethyl ether, diethylene glycol monobutyl ether, triethylene glycol monomethyl ether, dipropylene glycol monomethyl ether, dipropylene glycol monoethyl ether, tripropylene glycol monomethyl ether, etc.

Preferred are diethyl ether, methyl tert-butyl ether, dipropyl ether, diisopropyl ether, dibutyl ether, dihexyl ether, anisol, phenetole, butyl phenyl ether, methoxytoluene, dioxane, 2-methylfuran, tetrahydrofuran, tetrahydropyran, ethylene glycol dimethyl ether, ethylene glycol diethyl ether, ethylene glycol dibutyl ether, ethylene glycol monomethyl ether, ethylene glycol monoethyl ether, etc. More preferred are diethyl ether, methyl tert-butyl ether, anisol, dioxane, tetrahydrofuran, ethylene glycol monomethyl ether, ethylene glycol monoethyl ether, etc. Still more preferred are diethyl ether, methyl tert-butyl ether, anisol, dioxane, tetrahydrofuran, etc., and particularly preferred are dioxane and tetrahydrofuran.

The ketones are not particularly restricted, and ones having 3 to 6 carbon atoms are preferably used. As specific examples, there may be mentioned, for example, acetone, methyl ethyl ketone, methyl butyl ketone, methyl isobutyl ketone, etc. Preferred are acetone and methyl ethyl ketone, and most preferred is acetone.

The nitriles are not particularly restricted, and may be cyclic or acyclic, or saturated or unsaturated. However, saturated ones are preferably used. Generally, ones containing 2 to 20 carbon atoms, preferably 2 to 12 carbon atoms, and more preferably 2 to 8 carbon atoms are used.

As specific examples, there may be mentioned, for example, acetonitrile, propiononitrile, malononitrile, butyronitrile, isobutyronitrile, succinonitrile, valeronitrile, glutaronitrile, hexanenitrile, heptylcyanide, octylcyanide, undecanenitrile, dodecanenitrile, tridecanenitrile, pentadecanenitrile, stearonitrile, chloroacetonitrile, bromoacetonitrile, chloropropiononitrile, bromopropiononitrile, methoxyacetonitrile, methyl cyanoacetate, ethyl cyanoacetate, tolunitrile, benzonitrile, chlorobenzonitrile, bromobenzonitrile, cyanobenzoic acid, nitrobenzonitrile, anisonitrile, phthalonitrile, bromotolunitrile, methyl cyanobenzoate, methoxybenzonitrile, acetylbenzonitrile, naphthonitrile, biphenylcarbonitrile, phenylpropiononitrile, phenylbutyronitrile, methylphenylacetonitrile, diphenylacetonitrile, naphthylacetonitrile, nitrophenylacetonitrile, chlorobenzylcyanide, cyclopropanecarbonitrile, cyclohexanecarbonitrile, cycloheptanecarbonitrile, phenylcyclohexanecarbonitrile, tolylcyclohexanecarbonitrile, etc.

Preferred are acetonitrile, propiononitrile, butyronitrile, isobutyronitrile, succinonitrile, valeronitrile, methyl cyanoacetate, ethyl cyanoacetate, benzonitrile, tolunitrile and chloropropiononitrile. More preferred are acetonitrile, propiononitrile, butyronitrile and isobutyronitrile, and most preferred is acetonitrile.

As the amides, there may be mentioned, for example, formamide, N-methylformamide, N, N-dimethylformamide, N,N-dimethylacetoamide, N-methylpyrrolidone, etc.

As the sulfur-containing compounds, there may be mentioned, for example, dimethyl sulfoxide, sulfolane, etc.

As the fatty acids, there may be mentioned, for example, formic acid, acetic acid, propionic acid, etc. Preferred are formic acid and acetic acid, and particularly preferred is acetic acid.

Among the above water-soluble solvents, alcohols, ethers, ketones, nitriles are preferred, alcohols and ketones are more preferred, monohydric alcohols containing 1 to 3 carbon atoms and acetone are still more preferred, and ethanol is most preferred.

The water-soluble organic solvents mentioned above may be used singly or in the form of a mixed solvent composed of two or more species. Furthermore, they may also be favorably used in the form of mixed solvents in combination with water. From the viewpoint of liquid characteristics and/or washing effects, the use of a mixed solvent composed of a water-soluble organic solvent and water is preferred.

When a mixed solvent composed of a water-soluble organic solvent and water is used, the concentration of the water-soluble organic solvent contained in the mixed solvent is not particularly restricted but, from the viewpoint for obtaining favorable liquid characteristics and washing effects, it is preferably not less than about 5 w/w%, more preferably not less than about 7 w/w%, still more preferably not less than about 10 w/w%, particularly preferably not less than about 20 w/w%, and most preferably not less than about 30 w/w%.

In cases where the product is used for foods or drugs, for instance, ethanol, 1,2-propanediol, polyethylene glycol (preferably polyethylene glycol having a molecular weight of 300 to 1000), glycerol and the like are suitable, and ethanol is particularly suitable. Needless to say, these solvents may be favorably used as a mixed solvent of two or more of them or as a mixed solvent in combination with water.

In the practice of the invention, a water-insoluble organic solvent may be used in combination with any of the water-soluble solvents mentioned above within the range that no substantial adverse effect will be caused. Such water-insoluble organic solvent includes hydrocarbons, fatty acid esters and the like, which are to be mentioned later herein.

The reduced coenzyme Q₁₀ to be used in the practice of the invention is one obtainable by reduction of oxidized coenzyme Q₁₀. More preferred is one obtainable by utilizing the reduction reaction according to the invention, which is to be mentioned later herein.

The purification method of the invention can be applied to reduced coenzyme Q₁₀ containing a relatively large amount of oxidized coenzyme Q₁₀, but it is especially effective in purifying high-purity reduced coenzyme Q₁₀ prepared by the reduction method to be mentioned later herein, or the like method. The reduced coenzyme Q₁₀ to be purified may be in the form of either crystals or oil as defined in the appended claims. The term "crystal(s)" of reduced coenzyme Q₁₀ as used herein also includes, within the meaning thereof, a solid obtainable by concentrating a reduced coenzyme Q₁₀-containing solution to dryness by distilling off the solvent, a solid resulting from solidification of oil of reduced coenzyme Q₁₀.

The water-soluble impurity to be removed in accordance with the present invention is not particularly restricted but includes, among others, the reducing agents used in the step of reducing oxidized coenzyme Q₁₀, which are to be mentioned later herein, and/or impurities derived from reducing agents. As the reducing agents and/or impurities derived from reducing agents, there may be mentioned, for example, hyposulfurous acid or salts thereof, and hydrogensulfites as byproducts derived from said hyposulfurous acid or salts thereof; ascorbic acid or related compounds thereof, and dehydroascorbic acid, 2,3-diketogulonic acid and oxalic acid as byproducts derived from said ascorbic acid or related compounds thereof; salts generated as byproducts from iron or zinc; and the like.

The method of washing is not particularly restricted but generally comprises bringing the crystals and/or oil of reduced coenzyme Q₁₀ into contact with the above-mentioned water-soluble organic solvent or the mixed solvent composed of the water-soluble organic solvent and water in a vessel since the amount of the solvent for washing can then be decreased. This contact is established by dispersing the crystals and/or oil of reduced coenzyme Q₁₀ in the water-soluble organic solvent or the mixed solvent composed of the water-soluble organic solvent and water, and particularly preferably suspending and/or emulsifying the crystals and/or oil of reduced coenzyme Q₁₀ in the water-soluble organic solvent or the mixed solvent composed of the water-soluble organic solvent and water to a sufficient extent.

Preferably, the washing is carried out under forced flowing. From the quality improvement viewpoint, the flowing is preferably effected by a power required for stirring per unit volume of generally not less than about 0.01 kW/m³, preferably not less than about 0.1 kW/m³, and more preferably not less than about 0.3 kW/m³. The above forced flowing is generally provided by rotation of a stirring blade(s). If the above flowing is attained, however, it is not always necessary to use a stirring blade(s). For example, the circulation of the liquid or the like procedure may be utilized.

The concentration of reduced coenzyme Q₁₀ during washing of crystals and/or oil of reduced coenzyme Q₁₀ is not particularly restricted but, from the viewpoint of attaining favorable liquid characteristics, the weight of reduced coenzyme Q₁₀ relative to the weight of the washing solvent at the time of completion of washing is preferably about not more than about 30 w/w%, more preferably not more than about 20 w/w%, still more preferably not more than about 15 w/w%, particularly preferably not more than about 13 w/w%, and most preferably not more than about 10 w/w%. By maintaining the above concentration, it becomes possible to realize more favorable washing with sufficient operationality for an industrial scale production. From the productivity viewpoint, the lower limit to that concentration is preferably about 1 w/w%, and more preferably about 2 w/w%.

The time of washing may vary depending on species of the water-soluble organic solvent or a mixed solvent composed of a water-soluble organic solvent and water, the proportion thereof, the amount of the washing solvent and so on, hence cannot be absolutely specified. Generally, however, the washing can be completed within a time not longer than 10 hours, preferably not longer than 5 hours, more preferably not longer than 2 hours, still more preferably not longer than 1 hour, particularly preferably not longer than 30 minutes, and most preferably not longer than 10 minutes.

The washing temperature may vary depending on the amount, composition and/or composition proportion of the solvent for washing (water-soluble organic solvent or a mixed solvent composed of a water-soluble organic solvent and water), the quality or purity of the reduced coenzyme Q₁₀ to be purified and so forth, hence cannot be absolutely specified. But when crystals of reduced coenzyme Q₁₀ is used, the upper limit is generally not higher than about 50°C, preferably not higher than about 45°C, more preferably not higher than about 40°C, and still more preferably not higher than about 35°C, and the lower limit is not lower than about -10°C, preferably not lower than about -5°C, still more preferably not lower than about 0°C. Generally, the washing can be favorably carried out within the range of about 0°C to 40°C. On the other hand, when oil of reduced coenzyme Q₁₀ is used, the lower limit is not lower than the melting temperature of reduced coenzyme Q₁₀, preferably not lower than about 40°C, more preferably not lower than about 45°C, still more preferably not lower than about 50°C, and particularly preferably not lower than about 60°C, and the upper limit is not higher than about 100°C, preferably not higher than about 90°C, more preferably not higher than about 80°C, and still more preferably not higher than about 70°C.

By washing crystals and/or oil of reduced coenzyme Q₁₀ in accordance with the above mentioned method, water-soluble impurities contained in the crystals and/or oil of reduced coenzyme Q₁₀ can be transferred into a water-soluble organic solvent or a mixed solvent composed of a water-soluble organic solvent and water, and thereby the water-soluble impurities can be removed from said the crystals and/or oil of reduced coenzyme Q₁₀.

In cases where crystals of reduced coenzyme Q₁₀ is purified, the reduced coenzyme Q₁₀ from which water-soluble impurities were removed can be recovered as a wet product, for example, by centrifugation, pressure filtration, vacuum filtration and/or the like procedure to remove the above-mentioned solvent used in the washing, if necessary followed by cake washing. Moreover, they can be recovered also as a dry product by further charging the wet product in a reduced pressure drier (vacuum drier) internally purged with an inert gas and drying the same under reduced pressure. The recovery as a dry product is preferred.

Furthermore, in cases where oil of reduced coenzyme Q₁₀ is purified by this method, it is possible to recover reduced coenzyme Q₁₀ as an oily form by separating a solvent used for washing from wash mixture (oil of reduced coenzyme Q₁₀ + a solvent(s) used for washing). On the other hand, it is also possible to recover reduced coenzyme Q₁₀ as a crystalline form by cooling the wash mixture as it is. The cooling temperature in the case where reduced coenzyme Q₁₀ is recovered as a crystalline form is not particularly restricted but is generally lower than about 50°C, preferably lower than about 48°C, more preferably lower than about 45°C, and still more preferably lower than about 40°C. The lower limit is the solidification temperature of the system and generally is not lower than about 0°C. In this case, the reduced coenzyme Q₁₀ from which water-soluble impurities were removed can be recovered despite the presence of water-soluble impurities in a wash mixture.

In cases where reduced coenzyme Q₁₀ was recovered as an oily form, it is also possible to favorably solidify the oil of reduced coenzyme Q₁₀ by contacting seed crystals (reduced coenzyme Q₁₀ own crystals) to the oil of reduced coenzyme Q₁₀, particularly by contacting seed crystals (reduced coenzyme Q₁₀ own crystals) to the oil of reduced coenzyme Q₁₀ at a temperature lower than the melting temperature. In this case, a solid may be obtained by forming the above oily product into a desired form after decreasing the temperature of the oily product to below the melting temperature thereof and contacting with the seed crystals. The contact with the crystals may be performed either before or after said formation from the oily product. The solidification temperature is not particularly restricted as long as it is lower than the melting temperature. Desirably, however, it is not lower than 0°C.

By recovering crystals (including solid) of reduced coenzyme Q₁₀ from oil of reduced coenzyme Q₁₀ in the above manner, the reagent and time losses can be avoided and crystals of reduced coenzyme Q₁₀ (including solid) can be favorably obtained in a high yield.

The weight content of water-soluble impurities in reduced coenzyme Q₁₀, when purified in the above manner, can be decreased generally to 0.15% or below, preferably 0.10% or below, more preferably 0.08% or below. Thus, reduced coenzyme Q₁₀ with very high quality can be obtained.

It is particularly preferable to perform the above purification procedure in a deoxygenated atmosphere. By doing so, it becomes possible to minimize the formation of oxidized coenzyme Q₁₀ as a byproduct and thus do the washing more efficiently.

The deoxygenated atmosphere can be attained by inert gas substitution, pressure reduction, boiling, or a combination of these. It is appropriate to perform at least inert gas substitution, namely to use an inert gas atmosphere. As the inert gas, there may be mentioned, for example, nitrogen gas, helium gas, argon gas, hydrogen gas, carbon dioxide gas or the like. Nitrogen gas is preferred, however.

Next, a method of synthesizing reduced coenzyme Q₁₀, which is suited for use in the practice of the invention, namely a method of reducing oxidized coenzyme Q₁₀ into reduced coenzyme Q₁₀, is described.

Reduced coenzyme Q₁₀ which can be used in the practice of the invention can be obtained as already mentioned hereinabove. They can be obtained by reducing oxidized coenzyme Q₁₀, such as an existing highly pure coenzyme Q₁₀, or a mixture of oxidized coenzyme Q₁₀ and reduced coenzyme Q₁₀ with a common reducing agent. First, the method of reducing oxidized coenzyme Q₁₀ is described.

Since reduced coenzyme Q₁₀ is apt to be oxidized by molecular oxygen to give oxidized coenzyme Q₁₀ as a byproduct, a solvent with high protective effect against oxidation is preferably used as the solvent in the step of reduction. Preferably, at least one species selected from among hydrocarbons, fatty acid esters, ethers, and nitriles is used as such solvent. Hydrocarbons are most preferred among them.

The hydrocarbons are not particularly restricted, but there may be mentioned, for example, aliphatic hydrocarbons, aromatic hydrocarbons, halogenated hydrocarbons, etc. Preferred are aliphatic hydrocarbons and aromatic hydrocarbons, and more preferred are aliphatic hydrocarbons.

The aliphatic hydrocarbons are not particularly restricted, and may be cyclic or acyclic, or saturated or unsaturated. However, generally they contain 3 to 20 carbon atoms, and preferably 5 to 12 carbon atoms.

As specific examples, there may be mentioned, for example, propane, butane, isobutane, pentane, 2-methylbutane, cyclopentane, 2-pentene, hexane, 2-methylpentane, 2,2-dimethylbutane, 2,3-dimethylbutane, methylcyclopentane, cyclohexane, 1-hexene, cyclohexene, heptane, 2-methylhexane, 3-methylhexane, 2,3-dimethylpentane, 2,4-dimethylpentane, methylcyclohexane, 1-heptene, octane, 2,2,3-trimethylpentane, isooctane, ethylcyclohexane, 1-octene, nonane, 2,2,5-trimethylhexane, 1-nonene, decane, 1-decene, p-menthane, undecane, dodecane, etc.

Among them, saturated aliphatic hydrocarbons having 5 to 8 carbon atoms are preferred, and preferably used are pentane, 2-methylbutane and cyclopentane, which have 5 carbon atoms (referred to as "pentanes"); hexane, 2-methylpentane, 2,2-dimethylbutane, 2,3-dimethylbutane, methylcyclopentane, cyclohexane, which have 6 carbon atoms (referred to as "hexanes"); heptane, 2-methylhexane, 3-methylhexane, 2,3-dimethylpentane, 2,4-dimethylpentane, methylcyclohexane, which have 7 carbon atoms (referred to as "heptanes"); octane, 2,2,3-trimethylpentane, isooctane, ethylcyclohexane, which have 8 carbon atoms (referred to as octanes); and a mixture of these. In particular, the above heptanes are still more preferred since they have a tendency to show a very high protective effect against oxidization, and heptane is most preferred.

The aromatic hydrocarbons are not particularly restricted, but generally they contain 6 to 20 carbon atoms, preferably 6 to 12 carbon atoms, and more preferably 7 to 10 carbon atoms. As specific examples, there may be mentioned, for example, benzene, toluene, xylene, o-xylene, m-xylene, p-xylene, ethylbenzene, cumene, mesitylene, tetralin, butylbenzene, p-cymene, cyclohexylbenzene, diethylbenzene, pentylbenzene, dipentylbenzene, dodecylbenzene, styrene, etc. Preferred are toluene, xylene, o-xylene, m-xylene, p-xylene, ethylbenzene, cumene, mesitylene, tetralin, butylbenzene, p-cymene, cyclohexylbenzene, diethylbenzene and pentylbenzene. More preferred are toluene, xylene, o-xylene, m-xylene, p-xylene, cumene and tetralin, and most preferred is cumene.

The halogenated hydrocarbons are not particularly restricted, and may be cyclic or acyclic, or saturated or unsaturated. However, acyclic halogenated hydrocarbons are preferably used. More preferred are chlorinated hydrocarbons and fluorinated hydrocarbons, and chlorinated hydrocarbons are particularly preferred. Additionally, ones containing 1 to 6 carbon atoms, preferably 1 to 4 carbon atoms, and more preferably 1 to 2 carbon atoms are favorably used.

As specific examples, there may be mentioned, for example, dichloromethane, chloroform, carbon tetrachloride, 1,1-dichloroethane, 1,2-dichloroethane, 1,1,1-trichloroethane, 1,1,2-trichloroethane, 1,1,1,2-tetrachloroethane, 1,1,2,2-tetrachloroethane, pentachloroethane, hexachloroethane, 1,1-dichloroethylene, 1,2-dichloroethylene, trichloroethylene, tetrachloroethylene, 1,2-dichloropropane, 1,2,3-trichloropropane, chlorobenzene, 1,1,1,2-tetrafluoroethane, etc.

Preferred are dichloromethane, chloroform, carbon tetrachloride, 1,1-dichloroethane, 1,2-dichloroethane, 1,1,1-trichloroethane, 1,1,2-trichloroethane, 1,1-dichloroethylene, 1,2-dichloroethylene, trichloroethylene, chlorobenzene and 1,1,1,2-tetrafluoroethane. More preferred are dichloromethane, chloroform, 1,2-dichloroethylene, trichloroethylene, chlorobenzene and 1,1,1,2-tetrafluoroethane.

The fatty acid esters are not particularly restricted, but there may be mentioned, for example, propionates, acetates, formates, etc. Preferred are acetates and formates, and more preferred are acetates. Ester functional groups thereof are not particularly restricted, but include alkyl esters having 1 to 8 carbon atoms, aralkyl esters having 7 to 12 carbon atoms. Preferred are alkyl esters having 1 to 6 carbon atoms, and more preferred are alkyl esters having 1 to 4 carbon atoms are used.

As the propionates, there may be mentioned, for example, methyl propionate, ethyl propionate, butyl propionate, isopentyl propionate, etc. Preferred is ethyl propionate.

As the acetates, there may be mentioned, for example, methyl acetate, ethyl acetate, propyl acetate, isopropyl acetate, butyl acetate, isobutyl acetate, sec-butyl acetate, pentyl acetate, isopentyl acetate, sec-hexyl acetate, cyclohexyl acetate, benzyl acetate, etc. Preferred are methyl acetate, ethyl acetate, propyl acetate, isopropyl acetate, butyl acetate, isobutyl acetate, sec-butyl acetate, pentyl acetate, isopentyl acetate, sec-hexyl acetate and cyclohexyl acetate. More preferred are methyl acetate, ethyl acetate, propyl acetate, isopropyl acetate, butyl acetate and isobutyl acetate. Most preferred is ethyl acetate.

As the formates, there may be mentioned, for example, methyl formate, ethyl formate, propyl formate, isopropyl formate, butyl formate, isobutyl formate, sec-butyl formate, pentyl formate, etc. Preferred are methyl formate, ethyl formate, propyl formate, butyl formate, isobutyl formate and pentyl formate, and most preferred is ethyl formate.

As the ethers and nitriles, there may be mentioned such ethers and nitriles as described hereinabove.

In selecting the solvent to be used from among those mentioned above, the following factors are preferably taken into consideration: such properties as boiling point and viscosity (e.g. boiling point (about 30 to 150°C at 1 atm) allowing appropriate warming for increasing the solubility and facilitating solvent removal by drying from wet crystals and/or solvent recovery from the filtrate after crystallization, etc.), an adequate melting point (not higher than about 20°C, preferably not higher than about 10°C, and more preferably not higher than about 0°C) hardly allowing solidification during handling at room temperature and upon cooling to a level below room temperature, and a low level of viscosity (about 10 cp or below at 20°C) . From the industrial operation viewpoint, those that are hardly volatile at ordinary temperature are preferred. Generally preferred are those having a boiling point of, for example, about 80°C or higher, and more preferably about 90°C or higher.

Among the above-mentioned solvents, those solvents which are low in miscibility with water are particularly preferably used as the solvent in carrying out the reduction reaction. They promote extraction/removal of the reducing agent (to be mentioned later) and impurities derived from the reducing agent into the aqueous phase and efficient purification/recovery of reduced coenzyme Q₁₀.

Reduced coenzyme Q₁₀ tends to become more resistant to oxidation as the concentration thereof in solution increases. Reduced coenzyme Q₁₀ is highly soluble in the solvents mentioned above and, from this viewpoint as well, the above solvents are suited for protection against oxidation. The concentration of reduced coenzyme Q₁₀ which is preferred for the protection against oxidation may vary depending on the solvent species, hence cannot be absolutely specified. Generally, however, the concentration of reduced coenzyme Q₁₀ in the solvents mentioned above is not lower than about 1 w/w%, preferably not lower than about 2 w/w%. The upper limit is not particularly restricted but, from the practical operationality viewpoint, it is about 400 w/w%, preferably about 200 w/w%, more preferably about 100 w/w%, and still more preferably about 50 w/w%.

Thus, when the above solvents are used, the undesirable oxygen-involved side reactions are minimized through the reduction step.

The reduction reaction can be carried out in one of the above solvents using, as the reducing agent, a metal hydride compound, iron (metallic iron or a salt-form iron), zinc (metallic zinc), hyposulfurous acid or a salt thereof, ascorbic acid or related compounds thereof, or the like.

The metal hydride compound is not particularly restricted but includes sodium borohydride, lithium aluminum hydride, etc. The amount of the metal hydride compound to be used may vary depending on the metal hydride compound species, hence cannot be absolutely specified. Generally, however, an amount of 1 to 3 times the theoretical hydrogen equivalent is suitable for carrying out the reduction reaction.

The reduction using iron or zinc is generally carried out using an acid. The acid is not particularly restricted but includes fatty acids such as acetic acid, sulfonic acids such as methanesulfonic acid, and inorganic acids such as hydrochloric acid and sulfuric acid, and the like. Inorganic acids are preferred, and sulfuric acid is more preferred.

The amount of iron to be used is not particularly restricted but an amount of about 1/5 by weight or more relative to the weight of reduced coenzyme Q₁₀ charged is appropriate for carrying out the reaction. The upper limit is not particularly restricted but, from the economic viewpoint, among others, it is about 2 times by weight or below. Iron may be used not only in a metallic form but also in the form of a salt such as iron(II) sulfate.

The amount of zinc to be used is not particularly restricted but an amount of about 1/10 by weight or more relative to the weight of reduced coenzyme Q₁₀ charged is appropriate for carrying out the reaction. The upper limit is not particularly restricted but, for the economic viewpoint, among others, it is not more than about 2 times by weight.

The hyposulfurous acid or the salt thereof is not particularly restricted but generally used in the form of an alkali metal salt (lithium salt, sodium salt, potassium salt, and the like), alkaline earth metal salt (magnesium salt, calcium salt, and the like), or ammonium salt, of hyposulfurous acid, for instance. Alkali metal salts, such as the lithium salt, sodium salt, and potassium salt, are preferred, and the sodium salt is more preferred.

The amount of the hyposulfurous acid or the salt thereof to be used is not particularly restricted but, generally, it is not less than about 1/5 by weight, preferably not less than about 2/5 by weight, and more preferably about 3/5 by weight, relative to the weight of reduced coenzyme Q₁₀ charged. A larger amount will not cause any trouble but is economically unfavorable. Thus, an amount not exceeding about 2 times by weight, preferably not exceeding the same weight, is employed. The reduction reaction can be appropriately carried out in an amount within the range from about 2/5 by weight to about the same weight.

The ascorbic acid and related compounds thereof are not particularly restricted, and include, for example, not only ascorbic acid, but also rhamno-ascorbic acid, arabo-ascorbic acid, gluco-ascorbic acid, fuco-ascorbic acid, glucohepto-ascorbic acid, xylo-ascorbic acid, galacto-ascorbic acid, gulo-ascorbic acid, allo-ascorbic acid, erythro-ascorbic acid, 6-desoxyascorbic acid, and the like related compounds, and may be ester forms or salts of these. Furthermore, these may be L-form, D-form or racemic form. More specifically, there may be mentioned, for example, L-ascorbic acid, L-ascorbyl palmitate, L-ascorbyl stearate, D-arabo-ascorbic acid, etc.

In producing the reduced coenzyme Q₁₀, any of the above-mentioned ascorbic acid and related compounds thereof may be favorably used. However, the water-soluble ones are favorably used in particular among the above-mentioned ascorbic acid or related compounds thereof in view of ease of separation from the generated reduced coenzyme Q₁₀, etc. And most preferred are a free form of L-ascorbic acid, D-arabo-ascorbic acid and the like in view of the ready availability, price, etc.

The amount of the ascorbic acid or a related compound thereof to be used is not particularly restricted but may be at such a level that is effective for converting oxidized coenzyme Q₁₀ to reduced coenzyme Q₁₀. Generally, it is used in an amount of not less than 1 mole, preferably not less than 1.2 moles, per mole of reduced coenzyme Q₁₀. The upper limit is not particularly restricted but, taking economic viewpoint into consideration, it is generally 10 moles, preferably 5 moles, and more preferably 3 moles, on the same basis.

The reducing agents mentioned above and/or compounds derivable therefrom are mostly soluble in water. When hyposulfurous acid or a salt thereof is used, for instance, a hydrogensulfite is formed as a byproduct. When ascorbic acid or a related compound thereof is used, dehydroascorbic acid is formed as a byproduct, and further 2,3-diketogulonic acid and oxalic acid are formed as byproducts from dehydroascorbic acid. Furthermore, when iron or zinc is used, salts (e.g. iron chloride or zinc chloride, which may be generated as a byproduct when hydrochloric acid is used) are formed as byproducts after reduction. As mentioned hereinabove, all of these reducing agents and/or compounds derived therefrom can be efficiently removed by using the purification method of the present invention, whereby high-quality reduced coenzyme Q₁₀ can be obtained.

Among the reducing agents mentioned above, zinc, hyposulfurous acid or a salt thereof, and ascorbic acid or a related compound thereof are more preferred, and hyposulfurous acid or a salt thereof (specifically, a hyposulfurous acid salt) and ascorbic acid or a related compound thereof are especially preferred, from the viewpoint of reducing ability, yield, quality or the like.

In the reduction reaction, such an alcohol as mentioned above and/or water can be appropriately used in combination. Combined use of water is suitable especially when iron, zinc or hyposulfurous acid or a salt thereof is used as the reducing agent. When a metal hydride compound or ascorbic acid or a related compound thereof is used as the reducing agent, an alcohol can be preferably used in combination. The combined use of water and/or an alcohol makes it possible to show the characteristics of these and contributes to improvements in reaction rate, yield and the like.

In the following, preferred modes of the reduction method are described in detail.

The reduction using the above-mentioned hyposulfurous acid or a salt thereof is preferably carried out in a mixed solvent system composed of water together with at least one organic solvent selected from among the above-mentioned hydrocarbons, fatty acid esters, ethers, and nitriles (preferably hydrocarbons, more preferably aliphatic hydrocarbons, still more preferably heptanes, and particularly preferably heptane).

On that occasion, the reaction is carried out generally at a pH of not higher than 7, preferably at pH 3 to 7, and more preferably at pH 3 to 6, from the yield and/or other viewpoint. The pH can be adjusted using an acid, for example a mineral acid such as hydrochloric acid or sulfuric acid, or a base, for example an alkali metal hydroxide such as sodium hydroxide.

In the reduction using hyposulfurous acid or a salt thereof, the amount of water to be used is not particularly restricted but may be such that a proper amount of hyposulfurous acid or a salt thereof, namely the reducing agent, can be dissolved. Generally, it is advisable to adjust the weight of the hyposulfurous acid or the salt relative to water to a level not more than about 30 w/w%, for instance, and preferably not more than about 20 w/w%. From the productivity viewpoint, among others, the amount of water is generally not less than about 1 w/w%, preferably not less than about 5 w/w%, and more preferably not less than about 10 w/w%.

The reduction using the above mentioned ascorbic acid or a related compound thereof can also be carried out using a solvent highly miscible with water as selected from among the above-mentioned hydrocarbons, fatty acid esters, ethers, and nitriles, in particular a highly water-miscible ether or nitrile, more specifically tetrahydrofuran, dioxane, acetonitrile, or the like. The use of such an alcohol and/or a ketone as mentioned above (preferably a highly water-miscible alcohol and/or ketone (specifically, a monohydric or dihydric (preferably monohydric) alcohol containing 1 to 5 carbon atoms, preferably 1 to 4 carbon atoms, more preferably 1 to 3 carbon atoms and/or such a ketone as acetone or methyl ethyl ketone)) is particularly preferred. Namely in the reduction reaction using ascorbic acid or a related compound, use of highly-miscible organic solvent is preferred.

The reduction using ascorbic acid or a related compound thereof can be carried out in the presence of a reaction promoter such as a basic substance or a hydrogensulfite salt in view of lowering the reaction temperature, shortening the reaction time and the like.

The above-mentioned basic substance is not particularly restricted but may be either an inorganic compound or an organic compound. The inorganic compound is not particularly restricted but includes hydroxides, carbonates and hydrogencarbonates of metals (preferably alkali metals, alkaline earth metals, etc.), ammonia, and the like. As typical examples thereof, there may be mentioned alkali metal hydroxides such as sodium hydroxide, alkali metal carbonates such as sodium carbonate, alkali metal hydrogencarbonates such as sodium hydrogencarbonate, alkaline earth metal carbonates such as magnesium carbonate, and the like. The organic compound is not particularly restricted but includes amines such as triethylamine, and the like.

Among the basic substances specifically mentioned above, weakly basic substances (weak base or weak alkali), for example such inorganic compounds as metal (preferably alkali metal, alkaline earth metal or the like) carbonates and hydrogencarbonates, and ammonia, and such organic compounds as triethylamine and like amines, are preferably used. Those weakly basic inorganic compounds mentioned above are more preferred.

As preferred species of the hydrogensulfite salt, there may be mentioned, for example, alkali metal hydrogensulfites such as sodium hydrogensulfite, and the like.

The amount of the above-mentioned reaction promoter is not particularly restricted but may be such that the reaction promoting effect of the reaction prompter can be produced to an expected extent (Namely, an effective amount). Taking economical viewpoint into consideration as well, it is generally not more than about 20 moles, preferably not more than about 10 moles, more preferably not more than about 5 moles, and still more preferably not more than about 2 moles, per mole of the ascorbic acid or a related compound thereof. The lower limit is not particularly restricted but generally is about 0.01 moles or higher, preferably about 0.05 moles or higher, more preferably about 0.1 moles or higher, and still more preferably about 0.2 moles or higher, on the same basis.

The reduction reaction is preferably carried out under forced flowing. The flowing is preferably effected by a power required for stirring per unit volume of generally not less than about 0.01 kW/m³, preferably not less than about 0.1 kW/m³, and more preferably not less than about 0.3 kW/m³. The above forced flowing is generally provided by rotation of a stirring blade (s) . If the above flowing is attained, however, it is not always necessary to use a stirring blade(s). For example, the circulation of the liquid or the like procedure may be utilized.

The reduction reaction temperature may vary depending on the reducing agent species and/or the amount thereof, hence cannot be absolutely specified. In the case of reduction using hyposulfurous acid or a salt thereof, for instance, the reaction can be carried out generally at about 100°C or below, preferably at about 80°C or below, and more preferably at about 60°C or below. The lower limit is the solidification temperature of the system. The reaction can be smoothly carried out at about 0 to about 100°C, preferably at about 0 to about 80°C, and more preferably at about 0 to about 60°C. The reduction using the ascorbic acid or a related compound thereof is carried out generally at about 30°C or above, preferably at about 40°C or above, and more preferably at about 50°C or above. The upper limit is the boiling point of the system. Generally, the reaction can be carried out at about 30 to about 150°C, preferably at about 40 to about 120°C, and more preferably at about 50 to about 100°C.

The reaction concentration is not particularly restricted but the weight of oxidized coenzyme Q₁₀ relative to the weight of the solvent is generally not less than about 1 w/w%, preferably not less than about 2 w/w%, more preferably not less than about 3 w/w%, still more preferably not less than about 5 w/w%, particularly preferably not less than about 10 w/w%, and most preferably not less than about 15 w/w%. The upper limit is not particularly restricted but generally is about 60 w/w%, preferably about 50 w/w%, more preferably about 40 w/w%, and still more preferably about 30 w/w%. Generally, the reaction may be favorably carried out at about 2 to about 30 w/w%, preferably at about 5 to about 30 w/w%, and more preferably at about 10 to about 30 w/w%.

The reduction reaction can be driven to completion generally within 48 hours, preferably within 24 hours, more preferably within 10 hours, and still more preferably within 5 hours.

The oil of reduced coenzyme Q₁₀ may also be obtained by removing the aqueous phase after reduction, in water, of oil of oxidized coenzyme Q₁₀ using the above-mentioned reducing agent.

In this case, the reduction is carried out generally at a temperature of about 45°C or above, preferably at about 48°C or above, and more preferably at about 50°C of above, although the temperature depends on the purity of reduced coenzyme Q₁₀ and other factors. The upper limit is the boiling point of the system and generally is about 100°C or below, preferably about 80°C or below, and more preferably about 60°C or below.

This method to reduce oil of oxidized coenzyme Q₁₀ in water makes it possible to synthesize reduced coenzyme Q₁₀ while avoiding the waste of time, the use of an expensive production apparatus and the increase in volume due to separation and concentration of the organic solvent.

The above reduction reaction and the after-treatment (separation of the organic phase) are very favorably carried out in a deoxygenated atmosphere, and it was also found that the operation under such atmosphere greatly contributed to the improvement in yield at the reduction reaction and the decrease of the amount of the reducing agent especially in the reduction reaction using hyposulfurous acid or a salt thereof. The deoxygenated atmosphere can be attained by inert gas substitution, pressure reduction, boiling, or a combination of these. It is appropriate to perform at least inert gas substitution, namely to use an inert gas atmosphere. The inert gas may be, for example, nitrogen gas, helium gas, argon gas, hydrogen gas, carbon dioxide gas, or the like. Nitrogen gas is preferred, however.

Now, the crystals of reduced coenzyme Q₁₀ to be used in the purification method of the present invention are described. For the practice of the invention, the crystals which may be available are those crystals obtainable by crystallization from or concentration to dryness of a solution containing reduced coenzyme Q₁₀, the already existing crystals of reduced coenzyme Q₁₀, and the like. The solid resulting from solidification of oil of reduced coenzyme Q₁₀ may also be used. Preferred are the crystals of reduced coenzyme Q₁₀ obtainable by crystallization or concentration to dryness. More preferred are the crystals of reduced coenzyme Q₁₀ obtainable by crystallization or concentration to dryness following reduction of oxidized coenzyme Q₁₀. Particularly preferred are the crystals of reduced coenzyme Q₁₀ obtainable by crystallization following reduction of oxidized coenzyme Q₁₀.

The solvent to be used in obtaining such crystals is not particularly restricted but includes hydrocarbons, fatty acid esters, ethers, alcohols, fatty acids, ketones, nitrogen-containing compounds (including nitriles and amides), sulfur-containing compounds, water, and the like.

As the hydrocarbons, fatty acid esters, ethers, alcohols, fatty acids, ketones, nitriles, amides, and sulfur-containing compounds, there may be mentioned such solvents as mentioned hereinabove.

As the nitrogen-containing compounds other than nitriles or amides, there may be mentioned, for example, nitromethane, triethylamine, pyridine, and the like.

For obtaining high-quality crystals of reduced coenzyme Q₁₀ while suppressing the undesirable oxygen-involving side reaction, it is preferred to use a solvent with high protective effect against such oxidation, namely at least one species selected from among the above-mentioned hydrocarbons, fatty acid esters, ethers, and nitriles. Among them, hydrocarbons and fatty acid esters are more preferred as such solvent, hydrocarbons are still more preferred, and heptanes are particularly preferred.

The use of such an alcohol and/or ketone as mentioned hereinabove is also preferred because crystals of reduced coenzyme Q₁₀ which have good slurry and crystal characteristics can be obtained when crystallization, which will be mentioned below as a method for recovering crystals, is utilized and such a solvent is used in the step of crystallization.

The crystallization of reduced coenzyme Q₁₀ can be carried out by the general crystallization procedure, for example cooling, concentration, solvent substitution, and use of a poor solvent, as used singly or in appropriate combination thereof. In particular, the cooling operation (crystallization by cooling) is preferably performed singly or in combination with some other operations.

For the crystallization of reduced coenzyme Q₁₀, it is very effective to purify and crystallize reduced coenzyme Q₁₀ with simultaneous removal of impurities contained in the reaction mixture or extract obtainable in the conventional manner or produced by the above-mentioned reduction method or the like. This makes it possible to remove coexisting impurities, in particular analogous compounds having a similar structure and generally not always easy to remove (specifically, reduced coenzyme Q₉, reduced coenzyme Q₈, reduced coenzyme Q₇, etc). Alcohols and/or ketones are particularly effective solvents for removing the compounds having similar structures as mentioned above.

Furthermore, when an alcohol and/or a ketone are (is) used and a small amount of water is allowed to coexist, the solubility of reduced coenzyme Q₁₀ can be properly reduced to give an increased yield. In addition, the slurry characteristics can be improved and, in particular, the solid-liquid separability (filterability) can be markedly improved, which is worthy of notice.

The mixing proportion between water and the alcohol and/or ketone solution may vary depending on the solvent species, hence cannot be absolutely specified. Any solvent substantially comprising the above-mentioned alcohol and/or ketone as a main component (s) can be used without any particular restriction. Namely, the proportion between the above alcohol and/or ketone in mixed solvent containing water has the lower limit of generally about 90 w/w%, preferably about 91 w/w%, more preferably about 92 w/w%, and still more preferably 93 w/w%, and the upper limit of about 99.5 w/w%, preferably about 99 w/w%, more preferably about 98 w/w%, and still more preferably about 97 w/w%. In general, the crystallization can be favorably carried out at about 90 to about 99.5 w/w%, and most preferably at about 93 to about 97 w/w%.

The crystallization temperature of reduced coenzyme Q₁₀ may vary depending on the crystallization solvent species and/or the method of crystallization, among others, hence cannot be absolutely specified. Generally, however, it is not higher than about 25°C, preferably not higher than about 20°C, more preferably not higher than about 15°C, and still more preferably not higher than about 10°C. The lower limit is the solidification temperature of the system. Generally, the crystallization is carried out at about 0 to about 25°C.

For minimizing immixture of various impurities into reduced coenzyme Q₁₀ obtained or for obtaining slurry having good properties, the amount of precipitation of crystals per unit time can be controlled in the step of crystallization. A preferred rate of crystallization per unit time is, for example, not higher than the rate at which about 50% of the whole amount of crystals crystallizes out per unit time (50% amount/hour), and preferably not higher than the rate at which about 25% of the whole amount of crystals crystallizes out (25% amount/hour) . The rate of cooling in cooling crystallization is generally not higher than about 40°C/hour, and preferably not higher than about 20°C/hour.

The crystallization of reduced coenzyme Q₁₀ is preferably carried out under forced flowing. For inhibiting the occurrence of supersaturation and effecting the nucleation and crystal growth smoothly, or from the quality improvement viewpoint, the flowing is preferably caused by a power required for stirring per unit volume of generally not less than about 0.01 kW/m³, preferably not less than about 0.1 kW/m³, and more preferably not less than about 0.3 kW/m³. The above forced flowing is generally provided by rotation of a stirring blade (s) . If the above flowing is attained, however, it is not always necessary to use a stirring blade(s). For example, the circulation of the liquid or the like procedure may be utilized.

For inhibiting the occurrence of supersaturation and effecting the nucleation and crystal growth smoothly in the step of crystallization, addition of seed crystals is preferred.

The crystallization concentration may vary depending on the crystallization solvent species and the method of crystallization, hence cannot be absolutely specified. For example, the weight of reduced coenzyme Q₁₀ relative to the crystallization solvent weight at the time of completion of crystallization is not more than about 15 w/w%, preferably not more than about 13 w/w%, and more preferably not more than about 10 w/w%. From the productivity viewpoint, the lower limit is generally not lower than about 1 w/w%, preferably not lower than about 2 w/w%, and more preferably not lower than about 5 w/w%. The crystallization can be favorably carried out generally at about 5 to about 10 w/w%.

The thus-obtained crystals of reduced coenzyme Q₁₀ can be recovered as a wet product, for example, by such a solid-liquid separation technique as centrifugation, pressure filtration, or vacuum filtration, if necessary followed by cake washing. Moreover, they can be recovered also as a dry product by further charging the wet product in a reduced pressure drier (vacuum drier) internally purged with an inert gas and drying the same under reduced pressure. The recovery in a dry form is preferred.

The oil of reduced coenzyme Q₁₀ to be used in the purification method of the present invention is oil resulting from melting of crystals of reduced coenzyme Q₁₀, or oil obtainable by concentrating a reduced coenzyme Q₁₀-containing solution at a temperature not lower than the melting temperature. Needless to say, the oil obtainable by concentrating a reduced coenzyme Q₁₀-containing solution, which is given by the above-mentioned reduction method, at a temperature not lower than melting temperature is also available.

The reduced coenzyme Q₁₀-containing organic phase to be used for obtaining oil of reduced coenzyme Q₁₀ is not particularly restricted but, for inhibiting the undesirable oxygen-involving side reaction to thereby obtain a high-quality oil of reduced coenzyme Q₁₀, it is preferably a solution in a solvent with high protective effect against such oxidation, namely in at least one solvent selected from among hydrocarbons, fatty acid esters, ethers, and nitriles. Among them, hydrocarbons and fatty acid esters are more preferred as the solvent. Hydrocarbons are still more preferred, and heptanes are most preferred. The reduced coenzyme Q₁₀-containing organic phase may be the above-mentioned solution or a concentrate obtainable by concentrating the solution in the general manner.

In concentrating the reduced coenzyme Q₁₀-containing organic phase, the concentration of said organic phase is carried out at a temperature equal to or higher than the melting temperature of the concentrate comprising reduced coenzyme Q₁₀ as a main component so that the coexisting solvent may be completely or nearly completely distilled off. As a result, an oily product of reduced coenzyme Q₁₀, from which solvents are completely or nearly completely removed, can be obtained. When the melting temperature is broad, the temperature is applicable as long as it is not lower than temperature at initiation of the melting.

In the practice of the invention, the temperature of concentration above for obtaining oil of reduced coenzyme Q₁₀ may vary depending on the amount of the coexisting organic solvent, hence cannot be absolutely specified. It is, however, for example, preferably not lower than about 40°C, more preferably not lower than about 45°C, still more preferably not lower than about 48°C, particularly preferably not lower than about 50°C, and most preferably about 60°C. The concentration can be favorably carried out generally within the range of about 40 to about 140°C, preferably about 40 to about 100°C, and more preferably about 50 to about 80°C, although the temperature depends on the solvent species and the amount thereof. The concentration is carried out at ordinary pressure or under reduced pressure.

The method mentioned above makes it possible to favorably obtain reduced coenzyme Q₁₀ as an oily product by completely distilling off the organic solvent without any stirring trouble even when the purity of reduced coenzyme Q₁₀ in the organic phase is, for example, not lower than about 80% by weight, preferably not lower than about 90% by weight, and more preferably not lower than 95% by weight.

When the oil of reduced coenzyme Q₁₀ is obtained by distilling off the solvent, content of the solvent in the oil of reduced coenzyme Q₁₀ is generally not higher than about 10% by weight, preferably not higher than about 5% by weight, and more preferably not higher than about 2% by weight, of the whole weight of the oil.

As described above, water-soluble impurities remaining in reduced coenzyme Q₁₀, in particular a reducing agent and/or impurities derived from the reducing agent, can be efficiently removed by the method of the present invention which is excellent in operationality.

The product reduced coenzyme Q₁₀ obtainable by the purification method of the invention is of very high quality, and the weight of water-soluble impurities contained in the reduced coenzyme Q₁₀ is expected to be 0.15% or lower, preferably 0.10% or lower, or more preferably 0.08% or lower.

### BEST MODE FOR CARRYING OUT THE INVENTION

The following examples illustrate the present invention further in detail.

In the examples, the content of L-ascorbic acid was determined by HPLC, and the sodium hyposulfite and sodium hyposulfite-derived impurity content was determined by ion chromatography for determination of the sodium content, which was then converted into the sodium hyposulfite content. It is to be understood, however, that the reducing agent and/or reducing agent-derived impurity contents shown will never indicate the limit of purification of reduced coenzyme Q₁₀ by the method of the invention.

### (Production Example 1)

Oxidized coenzyme Q₁₀ (100 g) and 60 g of L-ascorbic acid were added to 1000 g of ethanol, and the reduction reaction was carried out with stirring at 78°C. After 30 hours, the mixture was cooled to 50°C, and 400 g of ethanol and 100 g of water were added while maintaining the same temperature. This ethanol solution (containing 100 g of reduced coenzyme Q₁₀) was cooled to 2°C at a cooling rate of 10°C/hour with stirring (power for stirring: 0.3 kW/m³), and thereby white slurry was obtained. The slurry obtained was filtered under reduced pressure, and the wet crystals were dried under reduced pressure (20 to 40°C, 0.13 to 4 kPa (1 to 30 mmHg)) to give 101 g of dry white crystals (containing 3.2% of L-ascorbic acid and 0.36% of oxalic acid). All the operations other than the drying under reduced pressure were performed in a nitrogen atmosphere.

### (Example 1 and Comparative Example 1)

Ten-gram portions of the crystals of reduced coenzyme Q₁₀ (containing 3.2% of L-ascorbic acid and 0.36% of oxalic acid) as obtained in Production Example 1 were respectively added to 190 g each of aqueous ethanol solutions differing in ethanol content to give slurries. Each slurry was stirred at 25°C for 10 minutes. This slurry was filtered under reduced pressure, and the wet crystals were dried under reduced pressure (20 to 40°C, 0.13 to 4 kPa (1 to 30 mmHg)) to give dry white crystals. The contents of L-ascorbic acid and oxalic acid remaining in the thus-obtained crystals and the recovery percentage of reduced coenzyme Q₁₀ are shown in Table 1. All the operations other than the drying under reduced pressure were performed in a nitrogen atmosphere.

As Comparative Example 1, the result, obtained when 190 g of water is added in lieu of 190 g of ethanol in above Example 1, is also shown. In the Comparative Example 1, the liquid properties were very poor, for example crystals adhered to the wall surface, and the discharge operation was very difficult to carry out.

**Table 1**

| | Content of ethanol (%) | Content of L-ascorbic acid (%) | Content of oxalic acid (%) | Recovery percentage of reduced coenzyme Q₁₀ (%) |
|---|---|---|---|---|
| Example 1 | 10 | 0.07 | 0.05 | 99 |
| | 30 | 0.06 | 0.03 | 99 |
| | 50 | 0.06 | 0.02 | 99 |
| | 90 | 0.05 | 0.04 | 97 |
| Compar.Example 1 | 0 | 0.18 | 0.15 | 97 |

### (Example 2)

A 10-gram portion of the crystals of reduced coenzyme Q₁₀ (containing 3.2% of L-ascorbic acid and 0.36% of oxalic acid) as obtained in Production Example 1 was added to 190 g of ethanol to give slurry, which was stirred at 2°C for 10 minutes. This slurry was filtered under reduced pressure, and the wet crystals as dried under reduced pressure (20 to 40°C, 0.13 to 4 kPa (1 to 30 mmHg)) to give dry white crystals. All the operations other than the drying under reduced pressure were performed in a nitrogen atmosphere. In this case, the content of L-ascorbic acid remaining in the crystals was 0.06% and that of oxalic acid was 0.07%, and the recovery percentage of reduced coenzyme Q₁₀ was 96%.

### (Example 3 and Comparative Example 2)

A 10-gram portion of the crystals of reduced coenzyme Q₁₀ (containing 3.2% of L-ascorbic acid and 0.36% of oxalic acid) as obtained in Production Example 1 was converted to an oily form at 60°C. Thereto was added 190 g of a 30% (by weight) aqueous ethanol solution, and the mixture was stirred at the same temperature for 10 minutes. Thereafter, the mixture was cooled to 25°C to convert the oil to crystals. The resulting slurry was filtered under reduced pressure, and the wet crystals were dried under reduced pressure (20 to 40°C, 0.13 to 4 kPa (1 to 30 mmHg)) to give dry white crystals. The contents of L-ascorbic acid and oxalic acid remaining in the thus-obtained crystals and the recovery percentage of reduced coenzyme Q₁₀ are shown in Table 2. All the operations other than the drying under reduced pressure were performed in a nitrogen atmosphere.

As Comparative Example 2, the result, obtained when 190 g of water is added in lieu of 190 g of ethanol in above Example 3, is also shown. In the Comparative Example 2, the liquid properties were very poor, for example the oil of reduced coenzyme Q₁₀ would not be uniformly dispersed and, even after cooling, crystals adhered to the stirring blade, and the discharge operation was very difficult to carry out.

**Table 2**

| | Content of ethanol (%) | Content of L-ascorbic acid (%) | Content of oxalic acid (%) | Recovery percentage of reduced coenzyme Q₁₀(%) |
|---|---|---|---|---|
| Example 3 | 30 | 0.02 | 0. 03 | 99 |
| Compar. Examp le 2 | 0 | 0.43 | 0.19 | 97 |

### (Example 4)

A 10-gram portion of the crystals of reduced coenzyme Q₁₀ (containing 3.2% of L-ascorbic acid and 0.36% of oxalic acid) as obtained in Production Example 1 was added to 190 g of a 30% (by weight) acetone solution in water to give slurry, which was stirred at 25°C for 10 minutes. This slurry was filtered under reduced pressure, and the wet crystals were dried under reduced pressure (20 to 40°C, 0.13 to 4 kPa (1 to 30 mmHg)) to give dry white crystals. All the operations other than the drying under reduced pressure were performed in a nitrogen atmosphere. In this case, the content of L-ascorbic acid remaining in the crystals was 0.09% and that of oxalic acid was 0.04%, and the recovery percentage of reduced coenzyme Q₁₀ was 99%.

### (Production Example 2)

Oxidized coenzyme Q₁₀ (100 g) was dissolved in 1000 g of heptane at 25°C. Thereto was added gradually an aqueous solution, prepared as a reducing agent by adding 1000 ml of water to 100 g of sodium hyposulfite (purity at least 75%), for dissolution with stirring (power for stirring 0.3 kW/m³), and the reduction reaction was carried out at 25°C and at pH 4 to 6. After 2 hours of the reaction, the mixture was cooled to 2°C at a cooling rate of 10°C/hour with continued stirring (power for stirring 0.3 kW/m³), and thereby white slurry was obtained. All the above operations were carried out in a nitrogen atmosphere. The slurry obtained was filtered under reduced pressure, and the wet crystals were dried under reduced pressure (20 to 40°C, 0.13 to 4 kPa (1 to 30 mmHg)) to give 95 g of dry white crystals (containing 1.0% of sodium hyposulfite and sodium hyposulfite-derived impurities as expressed totally in terms of sodium hyposulfite). All the operations other than the drying under reduced pressure were performed in a nitrogen atmosphere.

### (Example 5 and Comparative Example 3)

A 10-gram portion of the crystals of reduced coenzyme Q₁₀ (containing 1.0% of sodium hyposulfite and sodium hyposulfite-derived impurities as expressed totally in terms of sodium hyposulfite) as obtained in Production Example 2 was purified by 1 hour of stirring in the same manner as in Example 1. The total amount of sodium hyposulfite and sodium hyposulfite-derived impurities remaining in the thus-obtained crystal and the recovery percentage of reduced coenzyme Q₁₀ are shown in Table 3.

As Comparative Example 3, the result, obtained when 190 g of water is added in lieu of 190 g of ethanol in above Example 5, is also shown. In the Comparative Example 3, the liquid properties were very poor, for example crystals adhered to the wall surface, and the discharge operation was very difficult to carry out.

**Table 3**

| | Content of ethanol (%) | The total amount of sodium hyposulfite and sodium hyposulfite-derived impurities remaining in reduced coenzyme Q₁₀ (%) | Recovery percentage of reduced coenzyme Q₁₀ (%) |
|---|---|---|---|
| Example 5 | 30 | 0.08 | 99 |
| Compar. Example 3 | 0 | 0.18 | 97 |

### (Example 6 and Comparative Example 4)

A 10-gram portion of the crystals of reduced coenzyme Q₁₀ (containing 1.0% of sodium hyposulfite and sodium hyposulfite-derived impurities as expressed totally in terms of sodium hyposulfite) as obtained in Production Example 2 was stirred in a 30% (by weight) aqueous ethanol solution at 60°C for 1 hour. Thereafter, the aqueous phase was removed at the same temperature to give oil of reduced coenzyme Q₁₀. This oil was dropped onto a plate (40°C) with reduced coenzyme Q₁₀ own crystals spread thereon to give a semispherical solid. The total amount of sodium hyposulfite and sodium hyposulfite-derived impurities remaining in the thus-obtained solid and the recovery percentage of reduced coenzyme Q₁₀ are shown in Table 4.

As Comparative Example 4, the result, obtained when 190 g of water is added in lieu of 190 g of ethanol in above Example 5, is also shown. In the Comparative Example 4, the oil of reduced coenzyme Q₁₀ would not be uniformly dispersed, and the liquid properties were thus very poor.

**Table 4**

| | Content of ethanol (%) | The total amount of sodium hyposulfite and sodium hyposulfite-derived impurities remaining in reduced coenzyme Q₁₀ (%) | Recovery percentage of reduced coenzyme Q₁₀ (%) |
|---|---|---|---|
| Example 6 | 30 | 0. 15 | 99 |
| compar. Example 4 | 0 | 0.35 | 97 |

### (Reference Example 1)

One-gram portions of reduced coenzyme Q₁₀ (reduced coenzyme Q₁₀/oxidized coenzyme Q₁₀ weight ratio 99.6/0.4) were respectively dissolved in 20 g each of various solvents specified in Table 5 at 25°C. After 24 hours of stirring in the air at 25°C, the reduced coenzyme Q₁₀/oxidized coenzyme Q₁₀ weight ratio in each solution was determined. The results thus obtained are shown in Table 5.

**Table 5**

| | R |
|---|---|
| Heptane | 99.1/0.9 |
| Hexane | 98.7/1.3 |
| Toluene | 98.8/1.2 |
| Chloroform | 98.9/1.1 |
| Ethylacetate | 98.9/1.1 |
| Methyl tert-butylether | 98.6/1.4 |
| Tetrahydrofuran | 98.5/1.5 |

| | |
|---|---|
| R:Reduced coenzyme Q₁₀/Oxidized coenzyme Q₁₀ weight ratio | |

### (Reference Example 2)

One-gram portions of reduced coenzyme Q₁₀ (reduced coenzyme Q₁₀/oxidized coenzyme Q₁₀ weight ratio 99.6/0.4) were respectively dissolved in 100 g each of various solvents specified in Table 6 at 35°C. After 24 hours of stirring in the air at 35°C, the reduced coenzyme Q₁₀/oxidized coenzyme Q₁₀ weight ratio in each solution was determined. The results thus obtained are shown in Table 6.

**Table 6**

| Solvent | R |
|---|---|
| Heptane | 96.7/3.3 |
| Ethylacetate | 96.4/3.6 |
| Acetonitrile | 96.0/4.0 |

| | |
|---|---|
| R:Reduced coenzyme Q₁₀/Oxidized coenzyme Q₁₀ weight ratio | |

### INDUSTRIAL APPLICABILITY

The invention, which has the constitution described hereinabove, makes it possible to conveniently and efficiently purify reduced coenzyme Q₁₀ by a method with good operationality. The method of the present invention is suitable for an industrial scale production, and thereby high quality reduced coenzyme Q₁₀ can be obtained.

## Claims

1. A method of purifying reduced coenzyme Q₁₀ which is obtainable by reduction of oxidized coenzyme Q₁₀,
wherein water-soluble impurities are removed from the reduced coenzyme Q₁₀,
wherein washing of the crystals and/or oil of the reduced coenzyme Q₁₀ comprises bringing crystals and/or oil of the reduced coenzyme Q₁₀ into contact with a water-soluble organic solvent or a mixed solvent composed of a water-soluble organic solvent and water in a vessel and wherein the washing of the crystals and/or oil of reduced coenzyme Q₁₀ is carried out in a state of dispersion of the crystals and/or oil of reduced coenzyme Q₁₀ in the water-soluble organic solvent or the mixed solvent composed of the water-soluble organic solvent and water and
wherein the crystals and/or oil of reduced coenzyme Q₁₀ are/is selected from the group consisting of: crystals obtainable by crystallization from or concentration to dryness of a solution containing reduced coenzyme Q₁₀; a solid resulting from solidification of oil of reduced coenzyme Q₁₀; an oil resulting from melting of crystals of reduced coenzyme Q₁₀; and an oil obtainable by concentrating a reduced coenzyme Q₁₀-containing solution at a temperature not lower than the melting temperature.

2. The method of purifying reduced coenzyme Q₁₀ according to Claim 1,
wherein the dispersion is caused in a state of forced flowing.

3. The method of purifying reduced coenzyme Q₁₀ according to any of Claims 1 or 2,
wherein the water-soluble organic solvent comprises at least one species selected from among alcohols, ketones, ethers, and nitriles.

4. The method of purifying reduced coenzyme Q₁₀ according to Claim 3,
wherein the water-soluble organic solvent is ethanol.

5. The method of purifying reduced coenzyme Q₁₀ according to any of Claims 1 to 4,
wherein the washing is carried out with a mixed solvent composed of an organic solvent and water.

6. The method of purifying reduced coenzyme Q₁₀ according to Claim 5,
wherein the washing is carried out with a mixed solvent having a water-soluble organic solvent content of not less than 5 w/w%.

7. The method of purifying reduced coenzyme Q₁₀ according to any of Claims 1 to 6,
wherein the water-soluble impurity is a reducing agent used for converting oxidized coenzyme Q₁₀ into reduced coenzyme Q₁₀ and/or an impurity derived from a reducing agent.

8. The method of purifying reduced coenzyme Q₁₀ according to Claim 7,
wherein the reducing agent and/or the impurity derived from a reducing agent are/is hyposulfurous acid or a salt thereof and/or an impurity derived from hyposulfurous acid or a salt thereof.

9. The method of purifying reduced coenzyme Q₁₀ according to Claim 7,
wherein the reducing agent and/or the impurity derived from a reducing agent are/is ascorbic acid or a related compound thereof and/or an impurity derived from ascorbic acid or a related compound thereof.

10. The method of purifying reduced coenzyme Q₁₀ according to Claim 9,
wherein the impurity derived from ascorbic acid or a related compound thereof is oxalic acid.

11. The method of purifying reduced coenzyme Q₁₀ according to any of Claims 1 to 10,
wherein the concentration of reduced coenzyme Q₁₀ during washing is not higher than 30 w/w% as expressed in terms of the weight of reduced coenzyme Q₁₀ relative to the weight of the solvent at the time of completion of the washing.

12. The method of purifying reduced coenzyme Q₁₀ according to any of Claims 1 to 11,
wherein reduced coenzyme Q₁₀ occurs as a form of crystals.

13. The method of purifying reduced coenzyme Q₁₀ according to Claim 12,
wherein the washing temperature is not higher than 50°C.

14. The method of purifying reduced coenzyme Q₁₀ according to any of Claims 1 to 13,
wherein reduced coenzyme Q₁₀ occurs as a form of oil and the washing temperature is not lower than the melting temperature of reduced coenzyme Q₁₀.

15. The method of purifying reduced coenzyme Q₁₀ according to Claim 14,
wherein the washing temperature is not lower than 40°C.

16. The method of purifying reduced coenzyme Q₁₀ according to Claim 14 or 15,
wherein crystals of reduced coenzyme Q₁₀ is recovered by cooling the solution obtainable after impurity removal from the oil of reduced coenzyme Q₁₀.

17. The method of purifying reduced coenzyme Q₁₀ according to Claim 14 or 15,
wherein crystals of reduced coenzyme Q₁₀ is recovered by contacting seed crystals to oil of reduced coenzyme Q₁₀ obtainable after impurity removal from said oil.

18. The method of purifying reduced coenzyme Q₁₀ according to any of Claims 1 to 17
wherein reduced coenzyme Q₁₀ is purified in a deoxygenated atmosphere.

## Patentansprüche

1. Verfahren zum Aufreinigen von reduziertem Coenzym-Q₁₀, das durch Reduzieren von oxidiertem Coenzym Q₁₀ erhalten werden kann,
wobei wasserlösliche Verunreinigungen aus dem reduzierten Coenzym-Q₁₀ entfernt werden,
wobei das Waschen von Kristallen und/oder Öl des reduzierten Coenzym-Q₁₀ umfasst:
das in Kontakt bringen der Kristalle und/oder des Öls des reduzierten Coenzym-Q₁₀ mit einem wasserlöslichen organischen Lösungsmittel oder einem gemischten Lösungsmittel, das aus einem wasserlöslichen organischen Lösungsmittel und Wasser zusammengesetzt ist, in einem Behälter, und
wobei das Waschen der Kristalle und/oder des Öls des reduzierten Coenzym-Q₁₀ in einem dispergierten Zustand der Kristalle und/oder des Öls des reduzierten Coenzym-Q₁₀ im wasserlöslichen organischen Lösungsmittel oder im gemischten Lösungsmittel, das aus einem wasserlöslichen organischen Lösungsmittel und Wasser zusammengesetzt ist, erfolgt, und
wobei die Kristalle und/oder das Öl des reduzierten Coenzym-Q₁₀ ausgewählt ist/sind aus der Gruppe bestehend aus:
Kristallen, die durch Kristallisieren aus oder durch Aufkonzentrieren zur Trockne einer Lösung, die reduziertes Coenzym-Q₁₀ enthält, erhalten werden können; einem Feststoff, der durch Verfestigen eines Öls des reduzierten Coenzym-Q10 entsteht; einem Öl, das durch Schmelzen von Kristallen des reduzierten Coenzym-Q10 entsteht; und einem Öl, das durch Aufkonzentrieren einer Lösung, die reduziertes Coenzym-Q₁₀ enthält, bei einer Temperatur, die nicht niedriger als die Schmelztemperatur ist, erhaltenen werden kann.

2. Verfahren zum Aufreinigen von reduziertem Coenzym-Q₁₀ gemäß Anspruch 1,
wobei die Dispersion in einem erzwungenen Fließzustand erhalten wird.

3. Verfahren zum Aufreinigen von reduziertem Coenzym-Q₁₀ gemäß Anspruch 1 oder 2,
wobei das wasserlösliche organische Lösungsmittel mindestens eine Spezies umfasst, die aus Alkoholen, Ketonen, Ethern und Nitrilen ausgewählt ist.

4. Verfahren zum Aufreinigen von reduziertem Coenzym-Q₁₀ gemäß Anspruch 3,
wobei das wasserlösliche organische Lösungsmittel Ethanol ist.

5. Verfahren zum Aufreinigen von reduziertem Coenzym-Q₁₀ gemäß einem der Ansprüche 1 bis 4,
wobei das Waschen mit einem gemischten Lösungsmittel, das aus einem wasserlöslichen organischen Lösungsmittel und Wasser zusammengesetzt ist, erfolgt.

6. Verfahren zum Aufreinigen von reduziertem Coenzym-Q₁₀ gemäß Anspruch 5,
wobei das Waschen mit einem gemischten Lösungsmittel mit einem Anteil an wasserlöslichem organischem Lösungsmittel von nicht weniger als 5 Gew.-% (w/w), erfolgt.

7. Verfahren zum Aufreinigen von reduziertem Coenzym-Q₁₀ gemäß einem der Ansprüche 1 bis 6,
wobei die wasserlösliche Verunreinigung ein Reduktionsmittel, das zum Überführen von oxidiertem Coenzym-Q₁₀ in reduziertes Coenzym-Q₁₀ verwendet wird und/oder eine von einem Reduktionsmittel ableitete Verunreinigung ist.

8. Verfahren zum Aufreinigen von reduziertem Coenzym-Q₁₀ gemäß Anspruch 7,
wobei das Reduktionsmittel und/oder die von einem Reduktionsmittel abgeleitete Verunreinigung Hyposchweflige Säure oder ein Salz davon und/oder eine von Hyposchwefliger Säure oder einem Salz davon abgeleitete Verunreinigung ist/sind.

9. Verfahren zum Aufreinigen von reduziertem Coenzym-Q₁₀ gemäß Anspruch 7,
wobei das Reduktionsmittel und/oder die von einem Reduktionsmittel abgeleitete Verunreinigung Ascorbinsäure oder eine damit verwandte Verbindung und/oder eine von Ascorbinsäure oder einer damit verwandten Verbindung abgeleitete Verunreinigung ist/sind.

10. Verfahren zum Aufreinigen von reduziertem Coenzym-Q₁₀ gemäß Anspruch 9,
wobei die von Ascorbinsäure oder einer damit verwandten Verbindung abgeleitete Verunreinigung Oxalsäure ist.

11. Verfahren zum Aufreinigen von reduziertem Coenzym-Q₁₀ gemäß einem der Ansprüche 1 bis 10,
wobei die Konzentration von reduziertem Coenzym-Q₁₀ während des Waschens nicht mehr als 30 Gew.-% (w/w) beträgt, ausgedrückt als Gewicht des reduzierten Coenzym-Q₁₀ relativ zum Gewicht des Lösungsmittels zum Zeitpunkt des Beendens des Waschens.

12. Verfahren zum Aufreinigen von reduziertem Coenzym-Q₁₀ gemäß einem der Ansprüche 1 bis 11,
wobei das reduzierte Coenzym-Q₁₀ in Form von Kristallen vorliegt.

13. Verfahren zum Aufreinigen von reduziertem Coenzym-Q₁₀ gemäß Anspruch 12,
wobei die Waschtemperatur nicht höher als 50°C ist.

14. Verfahren zum Aufreinigen von reduziertem Coenzym-Q₁₀ gemäß einem der Ansprüche 1 bis 13,
wobei das reduzierte Coenzym-Q₁₀ in Form von Öl vorliegt und die Waschtemperatur nicht niedriger als die Schmelztemperatur des reduzierten Coenzym-Q₁₀ ist.

15. Verfahren zum Aufreinigen von reduziertem Coenzym-Q₁₀ gemäß Anspruch 14,
wobei die Waschtemperatur nicht niedriger als 40°C ist.

16. Verfahren zum Aufreinigen von reduziertem Coenzym-Q₁₀ gemäß Anspruch 14 oder 15,
wobei die Kristalle des reduzierten Coenzym-Q₁₀ gewonnen werden, indem die Lösung, die nach dem Entfernen der Verunreinigungen aus dem Öl des reduzierten Coenzym-Q₁₀ erhalten werden kann, abgekühlt wird.

17. Verfahren zum Aufreinigen von reduziertem Coenzym-Q₁₀ gemäß Anspruch 14 oder 15,
wobei die Kristalle des reduzierten Coenzym-Q₁₀ gewonnen werden, indem Impfkristalle mit dem Öl des reduzierten Coenzym-Q₁₀, das nach dem Entfernen von Verunreinigungen aus dem Öl erhalten werden kann, in Kontakt gebracht werden.

18. Verfahren zum Aufreinigen von reduziertem Coenzym-Q₁₀ gemäß einem der Ansprüche 1 bis 17,
wobei das reduzierte Coenzym-Q₁₀ in einer sauerstofffreien Atmosphäre aufgereinigt wird.

## Revendications

1. Procédé de purification de coenzyme Q₁₀ réduite qui peut être obtenue par réduction de coenzyme Q₁₀ oxydée,
dans lequel des impuretés solubles dans l'eau sont enlevées de la coenzyme Q₁₀ réduite,
dans lequel un lavage des cristaux et/ou de l'huile de la coenzyme Q₁₀ réduite comprend la mise des cristaux et/ou de l'huile de la coenzyme Q₁₀ réduite en contact avec un solvant organique soluble dans l'eau ou un solvant mélangé composé d'un solvant organique soluble dans l'eau et d'eau dans un récipient et dans lequel le lavage des cristaux et/ou de l'huile de la coenzyme Q₁₀ réduite est effectué dans un état de dispersion des cristaux et/ou de l'huile de la coenzyme Q₁₀ réduite dans le solvant organique soluble dans l'eau ou dans le solvant mélangé composé du solvant organique soluble dans l'eau et d'eau, et
dans lequel les cristaux et/ou l'huile de la coenzyme Q₁₀ réduite sont sélectionnés dans le groupe se composant de : cristaux pouvant être obtenus par cristallisation ou concentration jusqu'à dessèchement d'une solution contenant la coenzyme Q₁₀ réduite ; un solide résultant d'une solidification d'huile de la coenzyme Q₁₀ réduite ; une huile résultant d'une fusion de cristaux de la coenzyme Q₁₀ réduite ; et une huile pouvant être obtenue par concentration d'une solution contenant la coenzyme Q₁₀ réduite à une température qui n'est pas inférieure à la température de fusion.

2. Procédé de purification de coenzyme Q₁₀ réduite selon la revendication 1,
dans lequel la dispersion est provoquée dans un état d'écoulement forcé.

3. Procédé de purification de coenzyme Q₁₀ réduite selon l'une quelconque des revendications 1 ou 2,
dans lequel le solvant organique soluble dans l'eau comprend au moins une espèce sélectionnée parmi des alcools, des cétones, des éthers et des nitriles.

4. Procédé de purification de coenzyme Q₁₀ réduite selon la revendication 3,
dans lequel le solvant organique soluble dans l'eau est de l'éthanol.

5. Procédé de purification de coenzyme Q₁₀ réduite selon l'une quelconque des revendications 1 à 4,
dans lequel le lavage est effectué avec un solvant mélangé composé d'un solvant organique et d'eau.

6. Procédé de purification de coenzyme Q₁₀ réduite selon la revendication 5,
dans lequel le lavage est effectué avec un solvant mélangé présentant une teneur en solvant organique soluble dans l'eau qui n'est pas inférieure à 5 % en poids.

7. Procédé de purification de coenzyme Q₁₀ réduite selon l'une quelconque des revendications 1 à 6,
dans lequel l'impureté soluble dans l'eau est un agent réducteur utilisé pour convertir la coenzyme Q₁₀ oxydée en coenzyme Q₁₀ réduite et/ou une impureté dérivée d'un agent réducteur.

8. Procédé de purification de coenzyme Q₁₀ réduite selon la revendication 7,
dans lequel l'agent réducteur et/ou l'impureté dérivée d'un agent réducteur sont/est un acide hyposulfureux ou un sel de celui-ci et/ou une impureté dérivée d'un acide hyposulfureux ou d'un sel de celui-ci.

9. Procédé de purification de coenzyme Q₁₀ réduite selon la revendication 7,
dans lequel l'agent réducteur et/ou l'impureté dérivée d'un agent réducteur sont/et un acide ascorbique ou un composé lié de celui-ci et/ou une impureté dérivée d'un acide ascorbique ou d'un composé lié de celui-ci.

10. Procédé de purification de coenzyme Q₁₀ réduite selon la revendication 9,
dans lequel l'impureté dérivée d'un acide ascorbique ou d'un composé lié de celui-ci est un acide oxalique.

11. Procédé de purification de coenzyme Q₁₀ réduite selon l'une quelconque des revendications 1 à 10,
dans lequel la concentration de la coenzyme Q₁₀ réduite au cours du lavage n'est pas supérieure à 30 % en poids, en étant exprimée en termes de poids de la coenzyme Q₁₀ réduite par rapport au poids du solvant à la fin du lavage.

12. Procédé de purification de coenzyme Q₁₀ réduite selon l'une quelconque des revendications 1 à 11,
dans lequel la coenzyme Q₁₀ réduite intervient sous forme de cristaux.

13. Procédé de purification de coenzyme Q₁₀ réduite selon la revendication 12,
dans lequel la température de lavage n'est pas supérieure à 50 °C.

14. Procédé de purification de coenzyme Q₁₀ réduite selon l'une quelconque des revendications 1 à 13,
dans lequel la coenzyme Q₁₀ réduite intervient sous forme d'huile et la température de lavage n'est pas inférieure à la température de fusion de la coenzyme Q₁₀ réduite.

15. Procédé de purification de coenzyme Q₁₀ réduite selon la revendication 14,
dans lequel la température de lavage n'est pas inférieure à 40 °C.

16. Procédé de purification de coenzyme Q₁₀ réduite selon la revendication 14 ou 15,
dans lequel des cristaux de la coenzyme Q₁₀ réduite sont récupérés par refroidissement de la solution pouvant être obtenue après l'enlèvement d'impuretés de l'huile de la coenzyme Q₁₀ réduite.

17. Procédé de purification de coenzyme Q₁₀ réduite selon la revendication 14 ou 15,
dans lequel des cristaux de la coenzyme Q₁₀ réduite sont récupérés en mettant des cristaux de germe en contact avec l'huile de la coenzyme Q₁₀ réduite pouvant être obtenue après l'enlèvement d'impuretés de ladite huile.

18. Procédé de purification de coenzyme Q₁₀ réduite selon l'une quelconque des revendications 1 à 17,
dans lequel la coenzyme Q₁₀ réduite est purifiée dans une atmosphère désoxygénée.
